# EUROPEAN PATENT SPECIFICATION

(11) **EP 1 721 590 B1**
(45) Date of publication and mention of the grant of the patent: **07.03.2012**
(21) Application number: 06016918.2
(22) Date of filing: 11.05.2001
(51) Int. Cl.: A61F 13/15, A61F 13/49

(54) **Method and apparatus for making prefastened and refastenable pant with desired waist and hip fit**
Verfahren und Vorrichtung, um eine bereits geschlossene und wiederverschliessbare Unterhose mit gewünschten Taillen- und Hüftmassen herzustellen
Procédé et dispositif de fabrication de couches-culottes dèja fixées ou refixables avec reglage souhaite à la ceinture et à la hanche.

(30) Priority: 16.05.2000 US 204495 P; 05.04.2001 US 827192
(43) Date of publication of application: 15.11.2006
(62) Divisional of application: 01933304.6
(73) Proprietor: KIMBERLY-CLARK WORLDWIDE, INC., Neenah, WI 54956 (US)
(72) Inventor: Popp, Robert Lee, Hortonville Wisconsin 54944 (US); Coenen, Joseph Daniel, Neenah Wisconsin 54956 (US); Kuen, David Arthur, Neenah Wisconsin 54956 (US); Olson, Christopher Peter, Neenah Wisconsin 54956 (US); Quereshi, Shawn Ahmed, Neenah Wisconsin 54956 (US)
(74) Representative: Davies, Christopher Robert

(56) References cited:
- EP-A- 0 689 816
- GB-A- 2 160 817
- US-A- 4 543 154
- US-A- 4 665 306
- US-A- 5 140 757
- US-A- 6 022 432

## Description

The present invention pertains to processes and apparatus for making garments, and more particularly to processes and apparatus for making prefastened and refastenable pants.

Prefastened and refastenable pants can be employed for many uses such as disposable absorbent garments. Examples of disposable absorbent garments include diapers, training pants or swim pants, feminine care products, adult incontinence products, or the like. The typical disposable absorbent garment is formed as a composite structure including an absorbent assembly disposed between a liquid permeable bodyside liner and a liquid impermeable outer cover. These components can be combined with other materials and features such as elastic materials and containment structures to form a product that is specifically suited to its intended purposes.

Prefastened and refastenable disposable absorbent garments can provide advantages over either conventional two-dimensional or three-dimensional products. Two-dimensional products are generally flat and provided in an unfastened configuration, but include fasteners to secure the product about the wearer. Three-dimensional products, in contrast, have closed sides so that the product has a unitary waist opening and two leg openings.

Prefastened and refastenable products can be applied and/or removed either like a conventional diaper or like a conventional training pant. For use as training pants, for example, there may be times when it would be useful to apply the product like a diaper. For instance, it might be more convenient to apply the product like a diaper when there is a desire not to remove the child's shoes. Because it is difficult to know when a particular mode of applying the garment will be needed, it is beneficial to have a garment that is adaptable to being used either as a diaper or as a pant. This is preferable to keeping both types of garments available. A product that can be applied like either a diaper or a pant permits the interior of the product to be easily checked without having to pull the product downward.

Prefastened and refastenable garments present new challenges for high speed manufacturing. The products must incorporate refastenable fasteners that are properly aligned and engaged. Improperly attached or aligned fasteners can lead to many product deficiencies, including machine waste and/or delay, improper fit, fastener delamination during use, fastener disengagement during use, skin irritation, or the like. Moreover, the manufacturing process should desirably be capable of making pants that provide proper fit in the waist and hip regions.

Thus, what is lacking and needed in the art are processes and apparatus for making prefastened disposable absorbent garments with refastenable fasteners, which processes and apparatus permit proper alignment of the fasteners while also yielding a product with desirable fit properties in the waist and hip regions.

A prior art process, having the features of the preamble of claim 1, is disclosed in US-6 022 432.

In response to the above-referenced unfulfilled need in the art, new processes and apparatus for making prefastened and refastenable pants have been discovered. The processes and apparatus allow for manufacturing a pant where folding operations are conducted parallel to the longitudinal centerline of the product. This significantly simplifies the manufacture of prefastened and refastenable garments. To obtain desirable fit properties in the waist and hip regions of fully assembled products, the pants are provided with an activatable retractive material. The process and apparatus provide for activation of the retractive material so that retraction occurs subsequent to folding.

According to the present invention, there is provided a process as claimed in claim 1, an apparatus as claimed in claim 10 and a part as claimed in claim 15.

The process and apparatus disclosed herein can provide efficient manufacture of pants having a waistband-to-hip circumference ratio (WHCR) at a 70 gram loading that is about 95 percent or less, such as about 70 to about 95 percent, and more particularly about 90 or less, such as about 75 to about 90 percent. The differential in circumferences results from activation and resulting retraction of the retractive material in the waist region, and particularly the waistband. The smaller circumference at the waistband can hold the pant up on the body when worn, especially when the pant is weighted down with water, urine, BM or other substances encountered during use.

The waistband-to-hip circumference ratio (WHCR) of a pant refers to the ratio of the circumference of the pant measured at the waistband (the "waistband circumference") to the circumference of the pant measured at the hip section (the "hip circumference"), measured at a specified loading and expressed as a percentage. The waistband peripherally surrounds the waist opening of the pant and is formed upon joining the front and back waist regions along refastenable seams. The hip section is disposed between the waistband and the leg openings of the pant and is also formed upon joining the front and back waist regions along the refastenable seams. Both the waistband and the hip section include portions of the front and back waist regions.

The WHCR is measured according to the WHCR procedure set forth hereinafter. In particular, the waistband and hip section circumferences can be measured perpendicular to the longitudinal centerline of the pant at a force of 70 grams. Unless specifically noted, reference herein to WHCR means measurements at a force of 70 grams. At significantly higher forces, for example about 2000 grams, the WHCR can be 100 percent so that the product is easy to raise up over the hips. Particularly, the maximum elongation in the waistband can be about the same as the maximum elongation in the hip section to enable the waistband of the pant to slide up over the hips. In a majority of individuals, including children, the hips have a larger circumference than the waist.

Thus, the waistband can desirably retain its ability to expand to the pre-activated circumference. This ability to expand can be provided by extensible and/or elastic elements provided in the waistband or by extensible and/or elastic retractive members. In particular embodiments, the retractive material can provide the ability of the waistband to expand to approximately the pre-activated circumference. The degree of extensibility and/or elasticity of the retractive material will depend upon the material properties of the selected retractive material. For example, some heat shrinkable films may not be able to fully elongate to the pre-activated length without substantially higher tension. It is noteworthy that products that achieve a narrower waistband by having narrower cut of materials in the upper part of the waist region are unlikely to be able to achieve suitable expansion of the waistband. Similarly, products that achieve a narrower waistband by overlapping greater portions of the upper part of the waist region for fastening are unlikely to be able to achieve suitable expansion of the waistband.

The retractive material can comprise any material adapted to retract upon activation, whether immediately upon activation or subsequently thereto. The retractive material can comprise elastomeric or nonelastomeric materials. Suitable nonelastomeric retractive materials can comprise without limitation polyether block amides (PEBAX) or the like, and laminates thereof. Suitable elastomeric retractive materials can comprise without limitation LYCRA® materials, elastomeric materials including latex rubber or synthetic urethanes, or the like, and laminates thereof. In particular embodiments, the retractive material can comprise an elastomeric material having an unstable state relative to some other stable and elastic state. In such embodiments, the retractive material can but need not have elastomeric properties in the unstable state.

The elastomers useful in retractive materials can be selected from the group consisting of elastomeric thermoplastic polymers. The physical structure of the elastomer can be strands, cast or blown film, any non-woven web of fiber of a desired thermoplastic polymer or a combination thereof. Suitable elastomeric thermoplastic polymers include styrene block copolymers such as, for example, those available under the trademark KRATON® from Shell Chemical Company of Houston, Texas USA. KRATON® block copolymers are available in several different formulations, a number of which are identified in U.S. Patents 4,663,220; 4,323,534; 4,834,738; 5,093,422; and 5,304,599.

Other exemplary elastomeric materials that can be used include polyurethane elastomeric materials such as, for example, those available under the trademark PELLATHANE® from Dow Chemical Company of Midland, Michigan USA or under the trademark ESTANE® from B.F. Goodrich & Company of Akron, Ohio USA or under the trademark MORTHANE® from Morton Thiokol Corporation; polyester elastomeric materials such as, for example, those available under the trade designation HYTREL from E.I Dupont de Nemours & Company of Wilmington, Delaware USA and those known as ARNITEL® from DSM of Sittard, Holland; and polymers from metallocene-based catalysis which are available under the name ENGAGE® from Dow Chemical Company of Midland, Michigan USA for polyethylene-based polymers and from Exxon Chemical Company of Baytown, Texas USA under the trade name ACHIEVE® for polypropylene-based polymers and EXACT® and EXCEED® for polyethylene-based polymers.

The retractive materials can be caused to retract or begin to retract by any activation mechanism appropriate for the selected type of retractive material. Suitable activation methods can include without limitation any means of applying energy to the retractive material, such as heating, electromagnetic radiation such as ultraviolet, infrared, microwave, or gamma radiation; compaction or compression of the retractive material; or the like. For particular materials, removal of a compaction or compression force may activate the retraction.

Upon activation the retractive material can be transformed from an unstable state to a stable state by the application of any form of energy or by any other convenient mechanism. The activation mechanism will depend upon the nature of the retractive material. The stable state need not be an absolute state; rather, it is required only that the state following activation be relatively more stable than the state preceding activation and that the state following activation be sufficiently stable for practical use.

In particular embodiments, the retractive material can comprise an elastomeric material which can be elongated from a stable state to an extended and unstable state. The elastomeric material can be temporarily maintained in the extended and unstable state by application of a compaction force. After a period of time measured from the application of the compaction force, or upon application of energy, the elastomeric material will retract from the extended and unstable state to its stable state. For purposes of the present invention, this form of elastomeric material in its extended and unstable state constitutes a retractive material and the compaction force constitutes activation of the retractive material. Examples of such elastomeric materials include without limitation polyether block amides (PEBAX), or the like.

Activation can occur at any point in the process, provided that retraction caused by the activation occurs subsequent to engagement of the fastening components. . Retraction can occur subsequent to engaging the fastening components without impacting alignment of the fastening components. . Prior to retraction, the unstretched circumferences of the waistband and hip section can be generally equal, which facilitates manufacture and proper engagement of the fastening components.

The side panels are folded and the fastening components are engaged with one another prior to activation and retraction. This allows the side panels to be folded parallel to the longitudinal centerline, which is a relatively easier manufacturing process than folding the product at an angle relative to the longitudinal centerline. In particular embodiments, activation and retraction of the retractive material occurs in a packaging device such as a product stacker.

The retractive material can be disposed at any location within the pant provided retraction of the retractive material will facilitate reduction of the waistband circumference. In particular embodiments, the retractive material can be located in the side panels, between the side panels such as on the absorbent chassis, or a combination of the two. The retractive material can function as another element of the pant, such as the outer cover, the side panels, the bodyside liner, an elastic waistband feature, a fastening component, a pant disposal device, another element of the absorbent chassis, or the like.

The retractive material can be disposed in one or both of the waist regions, the waistband alone, or the waistband and either or both of the hip section or the crotch region. Where the retractive material is used in both the waistband and hip section, activation of the retractive material desirably contributes to greater reduction of the waistband circumference than of the hip circumference. This can be accomplished by having a greater amount of retractive material in the waistband, by selectively activating the retractive material in the waistband to a greater extent than the retractive material in the hip section, by orienting the retractive material in the waistband to be more receptive to activation than in the hip section, by using different types of retractive materials with different retractive properties, by reducing retractive ability of an elastomer after activation such as by cutting or compressing, by providing lower resistance to retraction in the waistband than in the hip section as a whole, or the like. Examples of selective heat activation include applying a higher temperature air flow or a greater volume of heated air to the waistband as compared to the hip section. The retractive material can be oriented in a transverse direction within the pant so that retraction causes a reduction of the waistband circumference of the pant. The retractive material can be adapted to retract upon activation in one direction or in two or more directions.

The pant can be folded in half by a variety of mechanisms. Where the side panels are folded subsequent to folding the product in half, it may be desirable to maintain separation of the side panels and separation of the fastening components while the product is folded in half. Throughout the folding process, the waistband and hip circumferences can be equal, that is a WHCR of approximately 100 percent, or could alternatively be unequal. The fastening components can be engaged simultaneously or sequentially with folding of the pant.

The fastening components can comprise separate elements bonded to another component of the pant. Alternatively, the fastening components can comprise a portion of another element of the pant, such as the bodyside liner, the outer cover, separate side panels if employed, or the like. Thus, the fastening components can be located on the side panels, between the side panels such as on the absorbent chassis, or a combination of the two. The fastening components can have any desired shape, such as square, rectangular, round, curved, oval, irregularly shaped, or the like. Each fastening component can comprise a single fastening element or multiple fastening elements.

The fastening components can comprise any refastenable fasteners suitable for absorbent articles, such as adhesive fasteners, cohesive fasteners, mechanical fasteners, or the like. In particular embodiments the fastening components comprise mechanical fastening elements for improved performance. Suitable mechanical fastening elements can be provided by interlocking geometric shaped materials, such as hooks, loops, bulbs, mushrooms, arrowheads, balls on stems, male and female mating components, buckles, snaps, or the like. In particular embodiments, the fastening components and mating fastening components comprise hook-and-loop fastening elements. One skilled in the art will recognize that the shape, density and polymer composition of the hooks and loops may be selected to obtain the desired level of securement between the fastening components and the mating fastening components. A more aggressive hook material may comprise a material with a greater average hook height, a greater percentage of directionally-aligned hooks, or a more aggressive hook shape.

A refastenable fastening system allows for easy inspection of the interior of the pant-like product. If necessary, the fastening system also allows the pant to be removed quickly and easily. This is particularly beneficial when the pant contains messy excrement. For training pants, the caregiver can completely remove the pant-like product and replace it with a new one without having to remove the child's shoes and clothing. Refastenable fastening systems may be used with a wide variety of absorbent and non-absorbent products, including training pants, swim pants, diaper pants, incontinence garments, feminine care products, health care garments, apparel for institutional, industrial and consumer use, or other garments using mechanical or adhesive fasteners.

Absorbent articles are adapted to be worn adjacent to the body of a wearer to absorb and contain various exudates discharged from the body. The absorbent articles are desirably prefastened to provide a pant-like product for the user. The product can then be pulled on like a conventional training pant, and subsequently checked or removed with the ease of a diaper-like product. Moreover, the product may be applied like a diaper rather than like a pant. Supplemental releasable fastening means such as frangible point bonds may be employed to maintain the absorbent article in a pant configuration until the user intentionally disengages the fasteners.

Particular training pants suitable for use with the present invention are disclosed in U.S. Patent Application Serial No. 09/444,083, filed on November 22, 1999 (corresponding to PCT application WO 00/37009 published June 29, 2000) by A. Fletcher et al. and titled "Absorbent Articles With Refastenable Side Seams;". This reference describes various materials and methods for constructing training pants. Training pants can also be constructed using the methods and apparatus disclosed in U.S. Patent 4,940,464 issued July 10,1990 to Van Gompel et al.; and U.S. Patent 5,766,389 issued June 16, 1998 to Brandon et al.

### Definitions

Within the context of this specification, each term or phrase below will include the following meaning or meanings.

"Bonded" refers to the joining, adhering, connecting, attaching, or the like, of two elements. Two elements will be considered to be bonded together when they are bonded directly to one another or indirectly to one another, such as when each is directly bonded to intermediate elements.

"Comprising" is inclusive or open-ended and does not exclude additional, unrecited elements or method steps.

"Connected" refers to the joining, adhering, bonding, attaching, or the like, of two elements. Two elements will be considered to be connected together when they are connected directly to one another or indirectly to one another, such as when each is directly connected to intermediate elements.

"Disposable" refers to articles which are designed to be discarded after a limited use rather than being laundered or otherwise restored for reuse.

"Disposed," "disposed on," and variations thereof are intended to mean that one element can be integral with another element, or that one element can be a separate structure bonded to or placed with or placed near another element.

"Elastic," "elasticized" and "elasticity" mean that property of a material or composite by virtue of which it tends to recover its original size and shape after removal of a force causing a deformation.

"Elastomeric" refers to a material or composite which can be elongated by at least 25 percent of its relaxed length and which will recover, upon release of the applied force, at least 10 percent of its elongation. It is generally preferred that the elastomeric material or composite be capable of being elongated by at least 100 percent, more preferably by at least 300 percent, of its relaxed length and recover, upon release of an applied force, at least 50 percent of its elongation.

"Fabrics" is used to refer to all of the woven, knitted and nonwoven fibrous webs.

"Flexible" refers to materials which are compliant and which will readily conform to the general shape and contours of the wearer's body.

"Force" includes a physical influence exerted by one body on another which produces acceleration of bodies that are free to move and deformation of bodies that are not free to move. Force is expressed in grams per unit area.

"Graphic" refers to any design, pattern, or the like that is visible on an absorbent article.

"Hydrophilic" describes fibers or the surfaces of fibers which are wetted by the aqueous liquids in contact with the fibers. The degree of wetting of the materials can, in turn, be described in terms of the contact angles and the surface tensions of the liquids and materials involved. Equipment and techniques suitable for measuring the wettability of particular fiber materials or blends of fiber materials can be provided by a Cahn SFA-222 Surface Force Analyzer System, or a substantially equivalent system. When measured with this system, fibers having contact angles less than 90° are designated "wettable" or hydrophilic, while fibers having contact angles greater than 90° are designated "nonwettable" or hydrophobic.

"Integral" is used to refer to various portions of a single unitary element rather than separate structures bonded to or placed with or placed near one another.

"Inward" and "outward" refer to positions relative to the center of an absorbent article, and particularly transversely and/or longitudinally closer to or away from the longitudinal and transverse center of the absorbent article.

"Layer" when used in the singular can have the dual meaning of a single element or a plurality of elements.

"Liquid impermeable", when used in describing a layer or multi-layer laminate, means that a liquid, such as urine, will not pass through the layer or laminate, under ordinary use conditions, in a direction generally perpendicular to the plane of the layer or laminate at the point of liquid contact. Liquid, or urine, may spread or be transported parallel to the plane of the liquid impermeable layer or laminate, but this is not considered to be within the meaning of "liquid impermeable" when used herein.

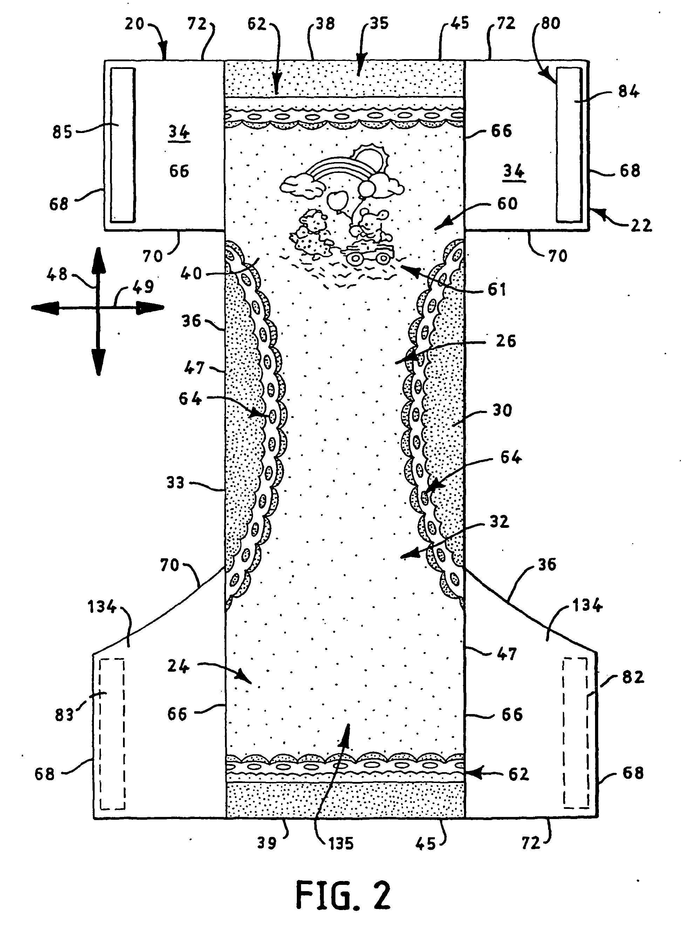

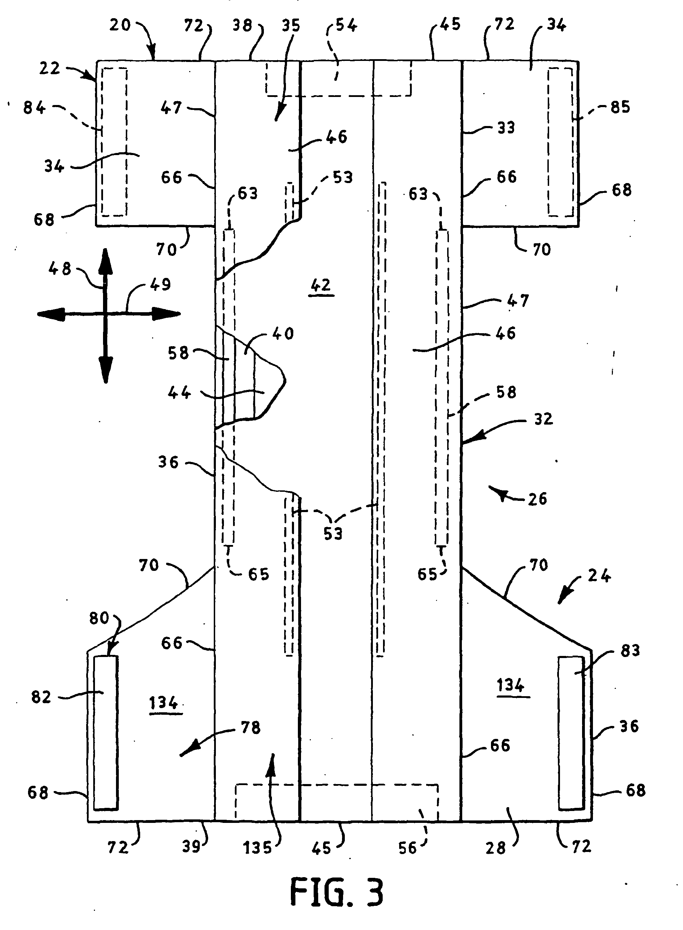

"Longitudinal" and "transverse" have their customary meaning, as indicated by the longitudinal and transverse axes depicted in Figures 2 and 3. The longitudinal axis lies in the plane of the article and is generally parallel to a vertical plane that bisects a standing wearer into left and right body halves when the article is worn. The transverse axis lies in the plane of the article generally perpendicular to the longitudinal axis. The article as illustrated is longer in the longitudinal direction than in the transverse direction.

"Member" when used in the singular can have the dual meaning of a single element or a plurality of elements.

"Nonwoven" and "nonwoven web" refer to materials and webs of material which are formed without the aid of a textile weaving or knitting process.

"Operatively joined," with reference to the attachment of an elastic member to another element, means that the elastic member when attached to or connected to the element, or treated with heat or chemicals, by stretching, or the like, gives the element elastic properties; and with reference to the attachment of a non-elastic member to another element, means that the member and element can be attached in any suitable manner that permits or allows them to perform the intended or described function of the joinder. The joining, attaching, connecting or the like can be either directly, such as joining either member directly to an element, or can be indirectly by means of another member disposed between the first member and the first element.

"Outer cover graphic" refers to a graphic that is directly visible upon inspection of the exterior surface of a garment, and for a refastenable garment is in reference to inspection of the exterior surface of the garment when the fastening system is engaged as it would be during use.

"Permanently bonded" refers to the joining, adhering, connecting, attaching, or the like, of two elements of an absorbent garment such that the elements tend to be and remain bonded during normal use conditions of the absorbent garment.

"Refastenable" refers to the property of two elements being capable of releasable attachment, separation, and subsequent releasable reattachment without substantial permanent deformation or rupture.

"Releasably attached," "releasably engaged" and variations thereof refer to two elements being connected or connectable such that the elements tend to remain connected absent a separation force applied to one or both of the elements, and the elements being capable of separation without substantial permanent deformation or rupture. The required separation force is typically beyond that encountered while wearing the absorbent garment.

"Rupture" means the breaking or tearing apart of a material; in tensile testing, the term refers to the total separation of a material into two parts either all at once or in stages, or the development of a hole in some materials.

"Stretch bonded" refers to an elastic member being bonded to another member while the elastic member is extended at least about 25 percent of its relaxed length. Desirably, the term "stretch bonded" refers to the situation wherein the elastic member is extended at least about 100 percent, and more desirably at least about 300 percent, of its relaxed length when it is bonded to the other member.

"Stretch bonded laminate" refers to a composite material having at least two layers in which one layer is a gatherable layer and the other layer is an elastic layer. The layers are joined together when the elastic layer is in an extended condition so that upon relaxing the layers, the gatherable layer is gathered.

"Surface" includes any layer, film, woven, nonwoven, laminate, composite, or the like, whether pervious or impervious to air, gas, and/or liquids.

"Tension" includes a uniaxial force tending to cause the extension of a body or the balancing force within that body resisting the extension.

"Thermoplastic" describes a material that softens when exposed to heat and which substantially returns to a nonsoftened condition when cooled to room temperature.

These terms may be defined with additional language in the remaining portions of the specification.

The above-mentioned and other features of the present invention and the manner of attaining them will become more apparent, and the invention itself will be better understood by reference to the following description and the accompanying drawings, wherein similar features in different figures have been given the same reference numeral.

Figure 1 illustrates a side view of a training pant suitable for use with the process and apparatus according to the present invention, where the fastening system is shown engaged on one side of the training pant and disengaged on the other side of the training pant.

Figure 2 illustrates a plan view of the training pant shown in Figure 1 in an unfastened, stretched and laid flat condition, and showing the surface of the training pant that faces away from the wearer.

Figure 3 illustrates a plan view similar to Figure 2, but showing the surface of the training pant that faces the wearer when the training pant is worn, and with portions cut away to show the underlying features.

Figure 4 schematically illustrates a flow diagram for manufacture of one embodiment of a pant according to the present invention.

Figure 5 illustrates a pant according to the present invention at three stages during manufacture.

Figure 6 illustrates an alternative pant according to the present invention at three stages during manufacture.

Figure 7 illustrates a side view of a tensile tester used to measure waistband-to-hip circumference ratio of the pants shown in Figures 1, 5 and 6.

Figure 8 illustrates a pant of the type shown in Figure 6 disposed on the tensile tester to measure the waistband circumference.

Figure 9 illustrates a pant of the type shown in Figure 6 disposed on the tensile tester to measure the hip circumference.

Figures 10 and 11 schematically illustrate pants in an unstretched condition and a fully stretched condition, respectively.

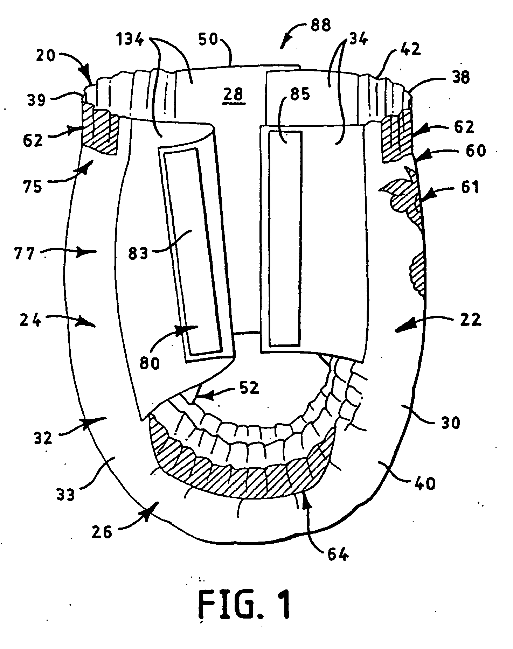

Figure 1 representatively illustrates one embodiment of training pant 20 in a partially fastened condition. The training pant 20 comprises an absorbent chassis 32 and a fastening system 80. The absorbent chassis 32 defines a front waist region 22, a back waist region 24, a crotch region 26 interconnecting the front and back waist regions, an inner surface 28 which is configured to contact the wearer, and an outer surface 30 opposite the inner surface which is configured to contact the wearer's clothing. With additional reference to Figures 2 and 3, the absorbent chassis 32 also defines a pair of transversely opposed side edges 36 and a pair of longitudinally opposed waist edges, which are designated front waist edge 38 and back waist edge 39. The front waist region 22 is contiguous with the front waist edge 38, and the back waist region 24 is contiguous with the back waist edge 39.

The illustrated absorbent chassis 32 comprises a rectangular composite structure 33, a pair of transversely opposed front side panels 34, and a pair of transversely opposed back side panels 134. The composite structure 33 and side panels 34 and 134 may be integrally formed or comprise two or more separate elements, as shown in Figure 1. The illustrated composite structure 33 comprises an outer cover 40, a bodyside liner 42 (Figures 1 and 3) which is connected to the outer cover in a superposed relation, an absorbent assembly 44 (Figure 3) which is located between the outer cover and the bodyside liner, and a pair of containment flaps 46 (Figure 3). The illustrated composite structure 33 has opposite linear end edges 45 that form portions of the front and back waist edges 38 and 39, and opposite linear side edges 47 that form portions of the side edges 36 of the absorbent chassis 32 (Figures 2 and 3). For reference, arrows 48 and 49 depicting the orientation of the longitudinal axis and the transverse axis, respectively, of the training pant 20 are illustrated in Figures 2 and 3.

With the training pant 20 in the fastened position as partially illustrated in Figure 1, the front and back waist regions 22 and 24 are joined together to define a three-dimensional pant configuration having a waist opening 50 and a pair of leg openings 52. The front waist region 22 comprises the portion of the training pant 20 which, when worn, is positioned on the front of the wearer while the back waist region 24 comprises the portion of the training pant which, when worn, is positioned on the back of the wearer. The crotch region 26 of the training pant 20 comprises the portion of the training pant which, when worn, is positioned between the legs of the wearer and covers the lower torso of the wearer. The front and back side panels 34 and 134 comprise the portions of the training pant 20 which, when worn, are positioned on the hips of the wearer.

The front waist region 22 of the absorbent chassis 32 includes the transversely opposed front side panels 34 and a front center panel 35 (Figures 2 and 3) positioned between and interconnecting the side panels. The back waist region 24 of the absorbent chassis 32 includes the transversely opposed back side panels 134 and a back center panel 135 (Figures 2 and 3) positioned between and interconnecting the side panels. The waist edges 38 and 39 of the absorbent chassis 32 are configured to encircle the waist of the wearer when worn and provide the waist opening 50 which defines a waist perimeter dimension. Portions of the transversely opposed side edges 36 in the crotch region 26 generally define the leg openings 52. The waist regions 22 and 24 jointly define a waistband 75 (Figures 1, 5, 6 and 8-11) that peripherally surrounds the waist opening 50 of the pant 20. The waist regions 22 and 24 also jointly define a hip section 77 (Figures 1, 5, 6 and 8-11) that encircles the pant 20 and is disposed between the waistband 75 and the leg openings 52.

The absorbent chassis 32 is configured to contain and/or absorb any body exudates discharged from the wearer. For example, the absorbent chassis 32 desirably although not necessarily comprises the pair of containment flaps 46 which are configured to provide a barrier to the transverse flow of body exudates. A flap elastic member 53 (Figure 3) is operatively joined with each containment flap 46 in any suitable manner as is well known in the art. The elasticized containment flaps 46 define an unattached edge which assumes an upright configuration in at least the crotch region 26 of the training pant 20 to form a seal against the wearer's body. The containment flaps 46 can be located along the transversely opposed side edges of the absorbent chassis 32, and can extend longitudinally along the entire length of the absorbent chassis or may only extend partially along the length of the absorbent chassis. Suitable constructions and arrangements for the containment flaps 46 are generally well known to those skilled in the art and are described in U.S. Patent 4,704,116 issued November 3, 1987 to Enloe.

To further enhance containment and/or absorption of body exudates, the training pant 20 desirably although not necessarily includes a front waist elastic member 54, a rear waist elastic member 56, and leg elastic members 58, as are known to those skilled in the art (Figure 3). The waist elastic members 54 and 56 can be operatively joined to the outer cover 40 and/or bodyside liner 42 along the opposite waist edges 38 and 39, and can extend over part or all of the waist edges, such that the waist elastic members are disposed in the waistband 75 in the fully assembled pant. The leg elastic members 58 are desirably operatively joined to the outer cover 40 and/or bodyside liner 42 along the opposite side edges 36 and positioned in the crotch region 26 of the training pant 20. The leg elastic members 58 can be longitudinally aligned along each side edge 47 of the composite structure 33. Each leg elastic member 58 has a front terminal point 63 and a back terminal point 65, which points represent the longitudinal ends of the elastic gathering caused by the leg elastic members. The front terminal points 63 can be located adjacent the longitudinally innermost parts of the front side panels 34, and the back terminal points 65 can be located adjacent the longitudinally innermost parts of the back side panels 134.

The flap elastic members 53, the waist elastic members 54 and 56, and the leg elastic members 58 can be formed of any suitable elastic material. As is well known to those skilled in the art, suitable elastic materials include sheets, strands or ribbons of natural rubber, synthetic rubber, or thermoplastic elastomeric polymers. The elastic materials can be stretched and adhered to a substrate, adhered to a gathered substrate, or adhered to a substrate and then elasticized or shrunk, for example with the application of heat; such that elastic constrictive forces are imparted to the substrate. In one particular embodiment, for example, the leg elastic members 58 comprise a plurality of dry-spun coalesced multifilament spandex elastomeric threads sold under the trade name LYCRA® and available from E. I. Du Pont de Nemours and Company, Wilmington, Delaware U.S.A.

In particular embodiments, the waist elastic members 54 and 56 can be formed of retractive materials. For example, the waist elastic members 54 and 56 can be formed of an elastomeric material that is adapted to retract upon activation by a source of heat.

The outer cover 40 desirably comprises a material that is substantially liquid impermeable, and can be elastic, stretchable or nonstretchable. The outer cover 40 can be a single layer of liquid impermeable material, but desirably comprises a multi-layered laminate structure in which at least one of the layers is liquid impermeable. For instance, the outer cover 40 can include a liquid permeable outer layer and a liquid impermeable inner layer that are suitably joined together by a laminate adhesive, ultrasonic bonds, thermal bonds, or the like. Suitable laminate adhesives, which can be applied continuously or intermittently as beads, a spray, parallel swirls, or the like, can be obtained from Findley Adhesives, Inc., of Wauwatosa, Wisconsin U.S.A., or from National Starch and Chemical Company, Bridgewater, New Jersey U.S.A. The liquid permeable outer layer can be any suitable material and desirably one that provides a generally cloth-like texture. One example of such a material is a 20 gsm (grams per square meter) spunbond polypropylene nonwoven web. The outer layer may also be made of those materials of which liquid permeable bodyside liner 42 is made. While it is not a necessity for outer layer to be liquid permeable, it is desired that it provides a relatively cloth-like texture to the wearer.

The inner layer of the outer cover 40 can be both liquid and vapor impermeable, or can be liquid impermeable and vapor permeable. The inner layer is desirably manufactured from a thin plastic film, although other flexible liquid impermeable materials may also be used. The inner layer, or the liquid impermeable outer cover 40 when a single layer, prevents waste material from wetting articles, such as bedsheets and clothing, as well as the wearer and caregiver. A suitable liquid impermeable film for use as a liquid impermeable inner layer, or a single layer liquid impermeable outer cover 40, is a 0.02 millimeter polyethylene film commercially available from Huntsman Packaging of Newport News, Virginia U.S.A. If the outer cover 40 is a single layer of material, it can be embossed and/or matte finished to provide a more cloth-like appearance. As earlier mentioned, the liquid impermeable material can permit vapors to escape from the interior of the disposable absorbent article, while still preventing liquids from passing through the outer cover 40. A suitable "breathable" material is composed of a microporous polymer film or a nonwoven fabric that has been coated or otherwise treated to impart a desired level of liquid impermeability. A suitable microporous film is a PMP-1 film material commercially available from Mitsui Toatsu Chemicals, Inc., Tokyo, Japan, or an XKO-8044 polyolefin film commercially available from 3M Company, Minneapolis, Minnesota U.S.A.

As shown in Figures 1 and 2, the training pant 20 and in particular the outer cover 40 desirably comprises one or more appearance-related components. Examples of appearance-related components include, but are not limited to, graphics; highlighting or emphasizing leg and waist openings in order to make product shaping more evident or visible to the user; highlighting or emphasizing areas of the product to simulate functional components such as elastic leg bands, elastic waistbands, simulated "fly openings" for boys, ruffles for girls; highlighting areas of the product to change the appearance of the size of the product; registering wetness indicators, temperature indicators, and the like in the product; registering a back label, or a front label, in the product; and registering written instructions at a desired location in the product.

The illustrated training pant 20, which is designed for use by young girls, includes a registered outer cover graphic 60. In this design, the registered graphic 60 includes a primary pictorial image 61, simulated waist ruffles 62, and simulated leg ruffles 64. The primary pictorial image 61 includes a rainbow, sun, clouds, animal characters, wagon and balloons. Any suitable design can be utilized for a training pant intended for use by young girls, so as to be aesthetically and/or functionally pleasing to them and the caregiver. The appearance-related components are desirably positioned on the training pant 20 at selected locations, which can be carried out using the methods disclosed in U.S. Patent 5,766,389 issued June 16, 1998 to Brandon et al. The primary pictorial image 61 is desirably positioned in the front waist region 22 along the longitudinal centerline of the training pant 20.

The liquid permeable bodyside liner 42 is illustrated as overlying the outer cover 40 and absorbent assembly 44, and may but need not have the same dimensions as the outer cover 40. The bodyside liner 42 is desirably compliant, soft feeling, and non-irritating to the child's skin. Further, the bodyside liner 42 can be less hydrophilic than the absorbent assembly 44, to present a relatively dry surface to the wearer and permit liquid to readily penetrate through its thickness. Alternatively, the bodyside liner 42 can be more hydrophilic or can have essentially the same affinity for moisture as the absorbent assembly 44 to present a relatively wet surface to the wearer to increase the sensation of being wet. This wet sensation can be useful as a training aid. The hydrophilic/hydrophobic properties can be varied across the length, width and depth of the bodyside liner 42 and absorbent assembly 44 to achieve the desired wetness sensation or leakage performance.

The bodyside liner 42 can be manufactured from a wide selection of web materials, such as synthetic fibers (for example, polyester or polypropylene fibers), natural fibers (for example, wood or cotton fibers), a combination of natural and synthetic fibers, porous foams, reticulated foams, apertured plastic films, or the like. Various woven and nonwoven fabrics can be used for the bodyside liner 42. For example, the bodyside liner can be composed of a meltblown or spunbonded web of polyolefin fibers. The bodyside liner can also be a bonded-carded web composed of natural and/or synthetic fibers. The bodyside liner can be composed of a substantially hydrophobic material, and the hydrophobic material can, optionally, be treated with a surfactant or otherwise processed to impart a desired level of wettability and hydrophilicity. For example, the material can be surface treated with about 0.45 weight percent of a surfactant mixture comprising Ahcovel N-62 from Hodgson Textile Chemicals of Mount Holly, North Carolina U.S.A. and Glucopan 220UP from Henkel Corporation of Ambler, Pennsylvania in an active ratio of 3:1. The surfactant can be applied by any conventional means, such as spraying, printing, brush coating or the like. The surfactant can be applied to the entire bodyside liner 42 or can be selectively applied to particular sections of the bodyside liner, such as the medial section along the longitudinal centerline.

A suitable liquid permeable bodyside liner 42 is a nonwoven bicomponent web having a basis weight of about 27 gsm. The nonwoven bicomponent can be a spunbond bicomponent web, or a bonded carded bicomponent web. Suitable bicomponent staple fibers include a polyethylene/polypropylene bicomponent fiber available from CHISSO Corporation, Osaka, Japan. In this particular bicomponent fiber, the polypropylene forms the core and the polyethylene forms the sheath of the fiber. Other fiber orientations are possible, such as multi-lobe, side-by-side, end-to-end, or the like. While the outer cover 40 and bodyside liner 42 can comprise elastomeric materials, it can be desirable in some embodiments for the composite structure to be generally inelastic, where the outer cover, the bodyside liner and the absorbent assembly comprise materials that are generally not elastomeric.

The absorbent assembly 44 (Figure 3) is positioned between the outer cover 40 and the bodyside liner 42, which components can be joined together by any suitable means such as adhesives, ultrasonic bonds, thermal bonds, or the like. The absorbent assembly 44 can be any structure which is generally compressible, conformable, non-irritating to the child's skin, and capable of absorbing and retaining liquids and certain body wastes. The absorbent assembly 44 can be manufactured in a wide variety of sizes and shapes, and from a wide variety of liquid absorbent materials commonly used in the art. For example, the absorbent assembly 44 can suitably comprise a matrix of hydrophilic fibers, such as a web of cellulosic fluff, mixed with particles of a high-absorbency material commonly known as superabsorbent material. In a particular embodiment, the absorbent assembly 44 comprises a matrix of cellulosic fluff, such as wood pulp fluff, and superabsorbent hydrogel-forming particles. The wood pulp fluff can be exchanged with synthetic, polymeric, meltblown fibers or short cut homofil bicomponent synthetic fibers and natural fibers. The superabsorbent particles can be substantially homogeneously mixed with the hydrophilic fibers or can be nonuniformly mixed. The fluff and superabsorbent particles can also be selectively placed into desired zones of the absorbent assembly 44 to better contain and absorb body exudates. The concentration of the superabsorbent particles can also vary through the thickness of the absorbent assembly 44. Alternatively, the absorbent assembly 44 can comprise a laminate of fibrous webs and superabsorbent material or other suitable means of maintaining a superabsorbent material in a localized area.

Suitable superabsorbent materials can be selected from natural, synthetic, and modified natural polymers and materials. The superabsorbent materials can be inorganic materials, such as silica gels, or organic compounds, such as crosslinked polymers, for example, sodium neutralized polyacrylic acid. Suitable superabsorbent materials are available from various commercial vendors, such as Dow Chemical Company located in Midland, Michigan U.S.A., and Stockhausen GmbH & Co. KG, D-47805 Krefeld, Federal Republic of Germany. Typically, a superabsorbent material is capable of absorbing at least about 15 times its weight in water, and desirably is capable of absorbing more than about 25 times its weight in water.

In one embodiment, the absorbent assembly 44 is generally rectangular in shape, and comprises a blend of wood pulp fluff and superabsorbent material. One preferred type of pulp is identified with the trade designation CR1654, available from U.S. Alliance, Childersburg, Alabama U.S.A., and is a bleached, highly absorbent sulfate wood pulp containing primarily soft wood fibers and about 16 percent hardwood fibers. As a general rule, the superabsorbent material is present in the absorbent assembly 44 in an amount of from about 5 to about 90 weight percent based on total weight of the absorbent assembly. The absorbent assembly 44 suitably has a density within the range of about 0.10 to about 0.35 grams per cubic centimeter. The absorbent assembly 44 may or may not be wrapped or encompassed by a suitable tissue wrap that may help maintain the integrity and/or shape of the absorbent assembly.

The absorbent chassis 32 can also incorporate other materials that are designed primarily to receive, temporarily store, and/or transport liquid along the mutually facing surface with absorbent assembly 44, thereby maximizing the absorbent capacity of the absorbent assembly. One suitable material is referred to as a surge layer (not shown) and comprises a material having a basis weight of about 50 to about 120 grams per square meter, and comprising a through-air-bonded-carded web of a homogenous blend of 60 percent 3 denier type T-256 bicomponent fiber comprising a polyester core/polyethylene sheath and 40 percent 6 denier type T-295 polyester fiber, both commercially available from Kosa Corporation of Salisbury, North Carolina U.S.A.

As noted previously, the illustrated training pant 20 has front and back side panels 34 and 134 disposed on each side of the absorbent chassis 32. These transversely opposed front side panels 34 and transversely opposed back side panels 134 can be permanently bonded along attachment lines 66 to the composite structure 33 of the absorbent chassis 32 in the respective front and back waist regions 22 and 24. More particularly, as shown best in Figures 2 and 3, the front side panels 34 can be permanently bonded to and extend transversely beyond the linear side edges 47 of the composite structure 33 in the front waist region 22, and the back side panels 134 can be permanently bonded to and extend transversely beyond the linear side edges of the composite structure in the back waist region 24. The side panels 34 and 134 may be attached using attachment means known to those skilled in the art such as adhesive, thermal or ultrasonic bonding. Alternatively, the side panels 34 and 134 can be formed as a portion of a component of the composite structure 33. For example, the side panels can comprise a generally wider portion of the outer cover, the bodyside liner, and/or another component of the absorbent chassis. The front and back side panels 34 and 134 can be permanently bonded together or be releasably attached to one another as illustrated by the fastening system 80.

The illustrated side panels 34 and 134 each define a distal edge 68 that is spaced from the attachment line 66, a leg end edge 70 disposed toward the longitudinal center of the training pant 20, and a waist end edge 72 disposed toward a longitudinal end of the training pant. The leg end edge 70 and waist end edge 72 extend from the side edges 47 of the composite structure 33 to the distal edges 68. The leg end edges 70 of the side panels 34 and 134 form part of the side edges 36 of the absorbent chassis 32. In the back waist region 24, the leg end edges 70 are desirably although not necessarily curved and/or angled relative to the transverse axis 49 to provide greater coverage toward the back of the pant as compared to the front of the pant. The waist end edges 72 are desirably parallel to the transverse axis 49. The waist end edges 72 of the front side panels 34 form part of the front waist edge 38 of the absorbent chassis 32, and the waist end edges 72 of the back side panels 134 form part of the back waist edge 39 of the absorbent chassis.

In particular embodiments for improved fit and appearance, the side panels 34 and 134 desirably have an average length dimension measured parallel to the longitudinal axis 48 that is about 20 percent or greater, and particularly about 25 percent or greater, of the overall length dimension of the absorbent article, also measured parallel to the longitudinal axis 48. For example, in training pants having an overall length dimension of about 54 centimeters, the side panels 34 and 134 desirably have an average length dimension of about 10 centimeters or greater, such as about 15 centimeters. While each of the side panels 34 and 134 extend from the waist opening 50 to one of the leg openings 52, the back side panels 134 have a continually decreasing length dimension moving from the attachment line 66 to the distal edge 68, as is best shown in Figures 2 and 3.

Each of the side panels 34 and 134 can include one or more individual, distinct pieces of material. In particular embodiments, for example, each side panel 34 and 134 can include first and second side panel portions that are joined at a seam, or can include a single piece of material which is folded over upon itself (not shown).

The side panels 34 and 134 desirably although not necessarily comprise an elastic material capable of stretching in a direction generally parallel to the transverse axis 49 of the training pant 20. Suitable elastic materials, as well as one process of incorporating elastic side panels into a training pant, are described in the following U.S. Patents: 4,940,464 issued July 10, 1990 to Van Gompel et al.; 5,224,405 issued July 6, 1993 to Pohjola; 5,104,116 issued April 14, 1992 to Pohjola; and 5,046,272 issued September 10, 1991 to Vogt et al. In particular embodiments, the elastic material comprises a stretch-thermal laminate (STL), a neck-bonded laminate (NBL), a reversibly necked laminate, or a stretch-bonded laminate (SBL) material. Methods of making such materials are well known to those skilled in the art and described in U.S. Patent 4,663,220 issued May 5, 1987 to Wisneski et al.; U.S. Patent 5,226,992 issued July 13, 1993 to Mormon; and European Patent Application No. EP 0 217 032 published on April 8, 1987 in the names of Taylor et al. Alternatively, the side panel material may comprise other woven or nonwoven materials, such as those described above as being suitable for the outer cover 40 or bodyside liner 42, or stretchable but inelastic materials.

In particular embodiments, one or more of the side panels 34 and 134 can be formed of retractive materials. For example, the side panels 34 and 134 can be formed of an elastomeric material that is adapted to retract upon activation by a source of heat.

The illustrated training pant 20 includes a fastening system 80 for refastenably securing the training pant about the waist of the wearer. The illustrated fastening system 80 includes first fastening components 82 and 83 that are adapted to refastenably connect to mating second fastening components 84 and 85. In one embodiment, one surface of each of the first fastening components 82 and 83 comprises a plurality of engaging elements that project from that surface. The engaging elements of the first fastening components 82 and 83 are adapted to repeatedly engage and disengage engaging elements of the second fastening components 84 and 85.

In one particular embodiment, the first fastening components 82 and 83 each comprise hook type fasteners and the second fastening components 84 and 85 each comprise complementary loop type fasteners. In another particular embodiment, the first fastening components 82 and 83 each comprise loop type fasteners and the second fastening components 84 and 85 each comprise complementary hook type fasteners. Alternatively, the fastening components can comprise interlocking similar surface fasteners; adhesive or cohesive fastening elements such as an adhesive fastener and an adhesive-receptive landing zone or material; or the like. Although the illustrated embodiments show the back waist region 24 overlapping the front waist region 22, which is convenient, the training pant 20 can also be configured so that the front waist region overlaps the back waist region.

Loop type fasteners typically comprise a fabric or material having a base or backing structure and a plurality of loop members extending upwardly from at least one surface of the backing structure. The loop material can be formed of any suitable material, such as acrylic, nylon or polyester, and can be formed by methods such as warp knitting, stitch bonding or needle punching. Suitable loop materials are available from Guilford Mills, Inc., Greensboro, North Carolina, U.S.A. under the trade designation No. 36549. Another suitable loop material can comprise a pattern un-bonded web as disclosed in U.S. Patent 5,858,515 issued January 12, 1999 to Stokes et al.

Hook type fasteners typically comprise a fabric or material having a base or backing structure and a plurality of hook members extending upwardly from at least one surface of the backing structure. In contrast to the loop type fasteners which desirably comprise a flexible fabric, the hook material advantageously comprises a resilient material to minimize unintentional disengagement of the fastener components as a result of the hook material becoming deformed and catching on clothing or other items. The term "resilient" as used herein refers to an interlocking material having a predetermined shape and the property of the interlocking material to resume the predetermined shape after being engaged and disengaged from a mating, complementary interlocking material. Suitable hook material can be molded or extruded of nylon, polypropylene or another suitable material. Suitable single-sided hook materials for the fastening components 82-85 are available from commercial vendors such as Velcro Industries B.V., Amsterdam, Netherlands or affiliates thereof, and are identified as Velcro HTH-829 with a uni-directional hook pattern and having a thickness of about 0.9 millimeters (35 mils) and HTH-851 with a uni-directional hook pattern and having a thickness of about 0.5 millimeters (20 mils); and Minnesota Mining & Manufacturing Co., St. Paul, Minnesota U.S.A., including specific materials identified as CS-600.

With particular reference to Figure 3, the first fastening components 82 and 83 are desirably although not necessarily disposed on the inner surface 28 of the training pant 20 in the back waist region 24. The first fastening components 82 and 83 are desirably positioned along the distal edges 68 of the back side panels 134, and abutting or adjacent to the waist end edge 72. In certain embodiments, for example, the first fastening components 82 and 83 can be located within about 2 centimeters, and more particularly within about 1 centimeter, of the distal edges 68, the waist end edges 72, and the leg end edges 70.

With particular reference to Figure 2, the second fastening components 84 and 85 are desirably although not necessarily disposed on the outer surface 30 of the training pant 20 in the front waist region 22. The second fastening components 84 and 85 are sized to receive the first fastening components 82 and 83 and are desirably positioned along the distal edges 68 of the front side panels 34, and abutting or adjacent to the waist end edge 72. In certain embodiments, for example, the second fastening components 84 and 85 can be located within about 2 centimeters, and more particularly within about 1 centimeter, of the distal edges 68, the waist end edges 72, and the leg end edges 70. Where the first fastening components 82 and 83 comprise loop type fasteners disposed on the inner surface 28 and the second fastening components 84 and 85 comprise hook type fasteners disposed on the outer surface 30, the first fastening components can be sized larger than the second fastening components to ensure coverage of the rigid, outwardly-directed hooks.

The fastening components 82-85 can be adhered to the side panels 34 and 134 by any means known to those skilled in the art such as adhesive bonds, sonic bonds or thermal bonds. In an alternative embodiment, the training pant 20 includes only a single second fastening component disposed in the front waist region 22 for refastenably connecting the first fastening components 82 and 83 (not shown). In a further alternative embodiment, the fastening components can comprise integral portions of the waist regions. For instance, one of the elastomeric front or back side panels can function as second fastening components in that they can comprise a material that is releasably engageable with fastening components disposed in the opposite waist region.

The fastening components are desirably rectangular, although they may alternatively be square, round, oval, curved or otherwise non-rectangularly shaped. In particular embodiments, each of the fastening components 82-85 defines a length dimension aligned generally parallel with the longitudinal axis 48 of the training pant 20 and a width dimension aligned generally parallel with the transverse axis 49 of the training pant. For a child of about 9 to about 15 kilograms (20-30 pounds), for example, the length dimension of the fastening components is desirably from about 5 to about 13 centimeters, such as about 10 centimeters, and the width dimension is desirably from about 0.5 to about 3 centimeters, such as about 1 centimeter. With particular embodiments, the fastening components can have a length-to-width ratio of about 2 or greater, such as about 2 to about 25, and particularly about 5 or greater, such as about 5 to about 8. For other embodiments such as for adult products, it may be desirable for one or more of the fastening components to comprise a plurality of relatively smaller fastening elements. In that case, a fastening component or individual fastening elements may have an even smaller length-to-width ratio, for example, of about 2 or less, and even about 1 or less.

When the fastening components 82-85 are releasably engaged, the side edges 36 of the absorbent chassis 32 in the crotch region 26 define the leg openings 52, the waist edges 38 and 39 of the absorbent chassis, including the waist end edges 72 of the side panels, define the waist opening 50, and the waist regions 22 and 24 jointly define a waistband 75 and hip section 77. For improved formation of the leg openings 52, it can be desirable in some embodiments for the front side panels 34 to be longitudinally spaced from the back side panels 134 (see Figures 2 and 3). For example, the front side panels 34 can be longitudinally spaced from the back side panels 134 by a distance equal to about 20 percent or greater, particularly from about 20 to about 60 percent, and more particularly from about 35 to about 50 percent, of the overall length dimension of the absorbent article.

When connected, the fastening components 82-85 form refastenable seams 88 (Figure 1) that desirably although not necessarily extend substantially the entire distance between the waist opening 50 and the leg openings 52. More specifically, the refastenable seams 88 can cover about 80 to 100 percent, and particularly about 90 to about 98 percent, of the distance between the waist opening 50 and each leg opening 52, which distance is measured parallel to the longitudinal axis 48. To construct the seams 88 to extend substantially the entire distance between the waist and leg openings 50 and 52, the fastening components 82-85 can be formed to cover about 80 to 100 percent, and more particularly about 90 to about 98 percent, of the distance between the waist end edge 70 and the leg end edge 72 of the side panels 34 and 134. In other embodiments, the fastening components can comprise a plurality of smaller fastening elements covering a smaller portion of the distance between the waist opening 50 and the leg openings 52, for example, about 20 to about 70 percent, but spaced apart to span a larger percentage of the distance between the waist opening and the leg openings.

For the refastenable seams 88 to be located at the sides of the wearer, it can be particularly desirable for the transverse distance between the first fastening components 82 and 83 to be substantially equal to the transverse distance between the second fastening components 84 and 85. The transverse distance between a set of fasteners is the distance measured parallel to the transverse axis 49 between the longitudinal centerlines of the fasteners, measured with the side panels 34 and 134 in an unstretched condition.

The various components of the training pant can be connected together by any means known to those skilled in the art such as, for example, adhesive, thermal and/or ultrasonic bonds. Desirably, most of the components are connected using ultrasonic bonding for improved manufacturing efficiency and reduced raw material costs. Suitable rotary ultrasonic horns are described in U.S. Patent 5,110,403 to Ehlert.

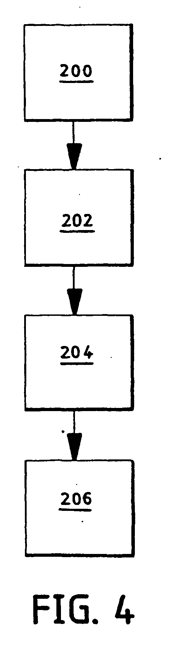

Figure 4 schematically illustrates a flow diagram for manufacture of one embodiment of a pant according to the present invention. The components of the pants 20 can be provided and bonded together in an assembly section 200, typically in the form of a continuous web of interconnected and partially assembled pants. The assembly section 200 can include a cutting mechanism which selectively cuts the web into discrete, partially assembled training pants.

The discrete training pants can then be folded at a folding station 202 using any suitable folding mechanism. The training pants 102 can be folded about a fold line generally bisecting the training pants. As such, the waist regions 22 and 24 of each training pant can be positioned in facing relationship with the side panels 34 and 134 extending laterally outward relative to the longitudinal axis 48 of the training pant. The fold line can extend in a lateral direction through the crotch region 26 of the training pant. Desirably, the discrete training pants are consistently folded about the fold line such that the front and back waist edges 38 and 39 of the training pants align with each other.

The opposed side panels 34 and 134 can also be folded in the folding section 202. The side panels 34 and 134 can desirably be folded parallel to the longitudinal centerline of the training pants so that at least portions of the first and second fastening components overlap with one another. The side panels 34 and 134 can alternatively be folded together in conjunction with engagement of the first and second fastening components 82-85 in a seaming section 204. The seaming section 204 forms the refastenable seams 88 of the pants 20. Suitable devices to inwardly fold the side panels 34 and 134 parallel to the longitudinal centerline can include folding boards, folding skis, paddles, fingers, vacuum devices, air blasts, mechanical devices with reciprocating motion such as tuckers, four-bar linkages, slide-crank mechanisms, or the like and combinations thereof.

The training pants 20 can be transported to an activation station 206, which follows the seaming section 204 as illustrated. The activation station 206 can comprise an activation source such as a heating unit to instigate retraction of the retractive material. The activation station 206 can comprise a separate stage of the manufacturing operation or can be incorporated into another stage. In particular embodiments, the activation station 206 can be combined with devices such as product stackers for packaging the pants 20. Suitable devices incorporating activation mechanisms are disclosed in U.S. Patents 4,640,726 issued February 3, 1987 to Sallee et al. and 4,663,106 issued May 5, 1987 to Pomplun et al.At least a portion of the retractive material is activated and caused to retract subsequent to engagement of the fastening components 82-85. While the waist elastic members 54 and 56 and side panels 34 and 134 were described as comprising retractive materials, other components of the training pant such as distinct elements or portions of the outer cover, bodyside liner, absorbent assembly or fastening components can alternatively or additionally comprise retractive materials.

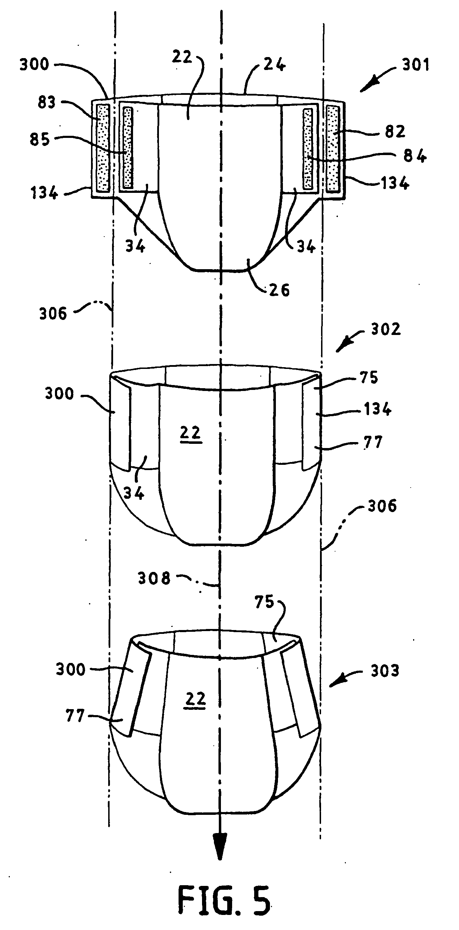

Figure 5 illustrates a training pant 300 at three stages during manufacture, denoted with reference numerals 301, 302 and 303. The training pant 300 is similar to the training pant 20 shown in Figure 1, although the front side panels 34 are narrower than the back side panels 134. The training pant 300 at stage 301 has been folded through the crotch region 26 to overlap the waist regions 22 and 24 and the hip regions. At stage 302, one or both pairs of side panels 34 and 134 of the training pant 300 can be folded along fold lines 306 which are parallel to the longitudinal centerline 308 of the training pant. With the fastening components 82-85 engaged, the waist regions 22 and 24 define the waistband 75 and hip section 77, both of which encircle the pant 300 between the crotch region 26 and the waist opening. The waistband 75 is contiguous with the waist opening 50 (Figure 1) while the hip section 77 is disposed between the waistband and the leg openings 52 (Figure 1). At stage 303, the retractive materials forming the waist elastic members 54 and 56, the side panels 34 and 134, and/or other components can be activated and the retractive materials allowed to retract. Retraction causes the pant 300 to have the desired WHCR.

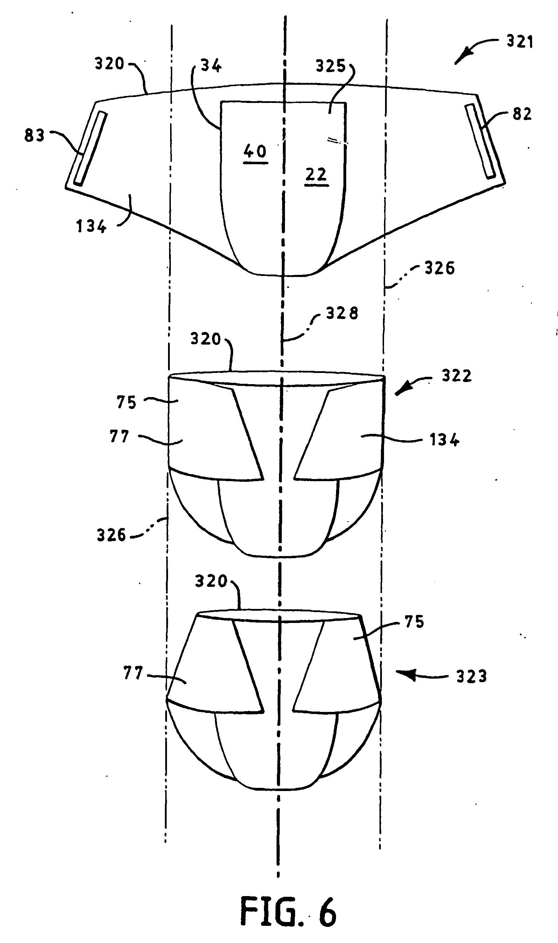

Figure 6 illustrates an alternative pant 320 at three stages during manufacture, denoted with reference numerals 321, 322 and 323. The pant 320 of this embodiment comprises integral back side panels 134 and first fastening components 82-83 oriented at an angle relative to the longitudinal centerline 328 of the training pant. The second fastening components 325 can comprise a portion or portions of the outer cover 40 or the front side panels 34. The stages 321-333 are similar to those described in relation to Figure 5. Despite the first fastening components 82-83 being oriented at an angle relative to the longitudinal centerline 328, the manufacturing process is significantly simplified by folding the back side panels 134 along fold lines 326 which are parallel to the longitudinal centerline 328. The training pant 320 is illustrated at stage 323 after activation and retraction of the retractive materials with the desired WHCR.

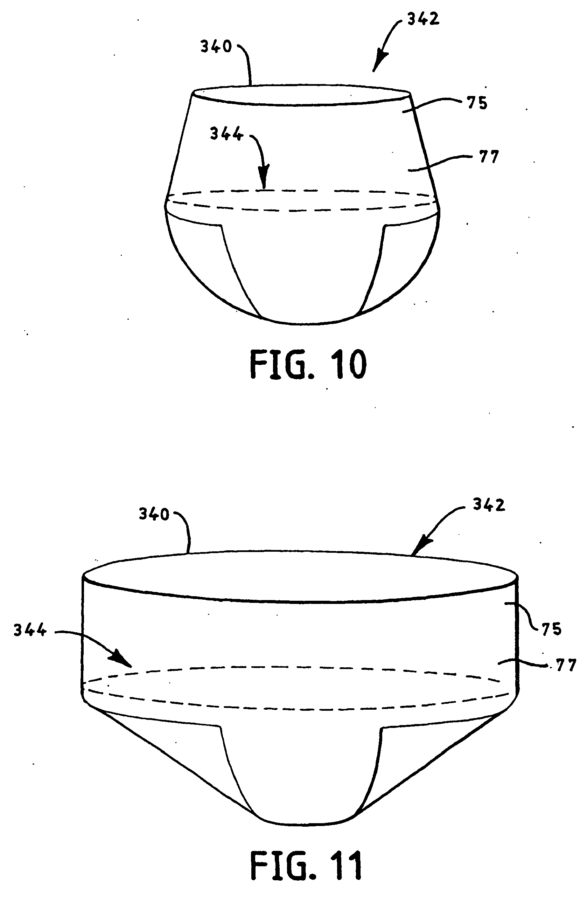

Figures 10 and 11 schematically illustrate a training pant 340 in an unstretched condition and a fully stretched condition, respectively. In the unstretched condition, (Figure 10), the circumference of the waistband 75, which is denoted at arrow 342, is smaller than the circumference of the hip section 77, which is denoted at arrow 344, to provide the desired WHCR. When the training pant 340 is stretched to a maximum point, for example, about 2000 grams (Figure 11), the waistband circumference 342 can but need not necessarily be substantially equal to the hip circumference 344.

### Waistband-to-hip Circumference Ratio WHCR Procedure

This procedure is a single-cycle tension bench test to measure waistband and hip circumferences of a test pant. The procedure measures waistband and hip circumferences under a minimum tension and also under a maximum tension. A test pant is cycled to a specific loading rather than to a fixed elongation/extension.

Data generated by this test method includes:
- Waistband circumference (mm) at an initial load of 70 g.
- Waistband circumference (mm) at a final (peak) load of 2000 g.
- Hip circumference (mm) at an initial load of 70 g.
- Hip circumference (mm) at a final (peak) load of 2000 g.

### 1. Overview

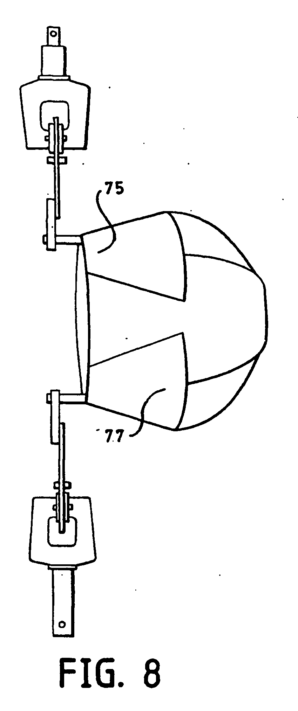

A pant is placed on the upper and lower pins in position to measure the waistband gage length, as shown in Figure 8. The gage length is selected for the waist opening of the pant being tested, so as to provide a tension of between 0 and 65 grams (g) when the pant is positioned for the test, prior to the start of the test. The term "tension" refers to the gram value measured by the load cells in the tensile tester.

The jaws are separated until a load of 70 grams of tension is attained, at which tension the gage length is recorded. Then the jaws continue to move apart until 2,000 grams of tension is reached, at which tension the gage length is again recorded. The standard test is one cycle per pant, although more can be used, and extension and tension data can be collected at 25 gram tension increments if desired. The circumference at a given tension may be calculated using the gage length and the circumference value(s) for the upper and lower pins. Desirably at least 3 pants are tested. The waistband circumference values at 70 grams tension from each pant tested are averaged to obtain an average initial waistband circumference, and the waistband circumference values at 2,000 grams tension from each pant tested are averaged to obtain an average final waistband circumference.

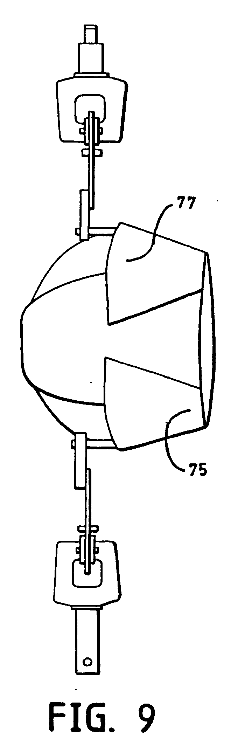

The procedure is repeated except that a fresh sample pant is placed on the upper and lower pins in position to measure values for the hip gage length, as shown in Figure 9. As with the waistband gage length, the standard test is one cycle per pant, although more can be used. Desirably at least 3 pants are tested. The hip circumference values at 70 grams tension from each pant tested are averaged to obtain an average initial hip circumference, and the hip circumference values at 2,000 grams tension from each pant tested are averaged to obtain an average final hip circumference. The sample products being tested can be randomized and separate samples are used to test each product parameter, thus eliminating position interactions.

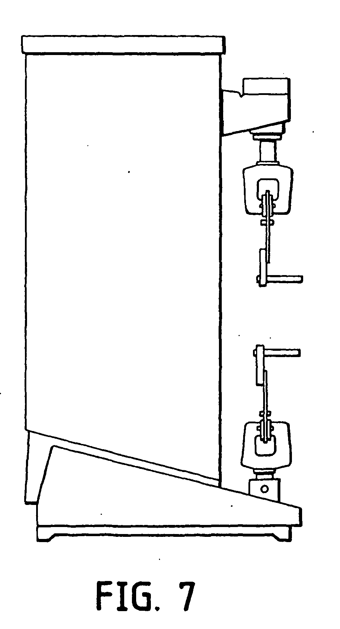

The waistband-to-hip circumference ratio of the pant at a given loading (tension level) is the average waist circumference at that loading divided by the average hip circumference at the same loading. Figure 7 illustrates a side view of a tensile tester used to measure waistband-to-hip circumference ratios of pants according to the present invention. Figure 8 illustrates a pant of the type shown in Figure 6 disposed on the tensile tester to measure the waistband circumference. Figure 9 illustrates a pant of the type shown in Figure 6 disposed on the tensile tester to measure the hip circumference.

### 2. Apparatus and Materials

| | |
|---|---|
| 2.1 | Constant Rate of Extension (CRE) tensile tester: MTS tensile tester model Synergie 200 Test Bed; available from MTS® Systems Corporation, Research Triangle Park, North Carolina USA. |
| 2.2 | Load cells: A suitable cell selected so the majority of the peak load values fall between the manufacturer's recommended ranges of load cell's full scale value; Model 100N available from MTS® Systems Corporation, Research Triangle Park, North Carolina USA. |
| 2.3 | Operating software and data acquisition system: MTS TestWorks® for Windows software version 3.10; available from MTS® Systems Corporation, Research Triangle Park, North Carolina USA. |
| 2.4 | Grips: pneumatic-action grips, top and bottom, identified as part number 2712-003 available from Instron Corporation, Canton, Massachusetts USA. |
| 2.5 | Grip faces: 25 by 75-mm (1 by 3-inch), suitable for holding pins. |
| 2.6 | Pins: rigid pins having a length of 6.3 centimeters (2.5 inch) and a knurled portion at one end for holding specimens, the knurled portion having an outside diameter of 6.4 millimeter (0.25 inch) and a length of 3.2 centimeters (1.25 inch). |
| 2.7 | Clips: 1.9 cm. wide by 0.95 cm. capacity (3/4" wide by 3/8" capacity) binder clips; part no. BTM00251 available from BT Office Products, Milwaukee, Wisconsin, USA. |

### 3. Conditioning

Conduct test in standard ASTM laboratory conditions: atmosphere of 23 ± 2°C (73.4 ± 3.6°F) and 50 ± 5% relative humidity. The products should be measured after they equilibrate to laboratory conditions.

### 4. Test Specimen

No preparation needed. The whole pant is tested.

### 5. Procedure

### Tensile Tester test conditions:

| | |
|---|---|
| Cross head speed: | 250 mm/min |
| Full scale load: | 4540 g |
| Gage length: | Appropriate starting gage length settings for both hip and waistband are those that will generate initial loads of between 0 and 65 g in a previously untested product |
| Go to load (cycle trigger): | 2000 g (or a maximum load value that can be experienced by the sample without causing the sample to tear or otherwise come apart) |
| Number of cycles: | 1 |
| Elongation stop: | 450 mm (200%) |
| Break sensitivity: | 75% |

| | |
|---|---|
| A. | Install pin assemblies as depicted in Figure 7. |
| B. | Using the tensile frame pushbutton controls for crosshead position, move pins so that the pant can be mounted on the pins without stretching the pant. Determine the gage length by measuring from the centerline of the first pin to the centerline of the second pin. Calibrate the software to this initial gage length. |
| C. | Place the waistband onto the knurled section of the top pin. Center one side of the pant on top of the pin. Use a single binder clip to hold the pant at the waist opening in place on the pin; do not stretch the pant during application of the clip. |
| D. | Click on ZERO to tare the load of the pant. Only tare the weight of the first pant for each sample population, not for each specimen. |
| E. | Place the waistband on the opposite side of the pant on the bottom pin and clip in place as for the first pin. Adjust pant so both top and bottom pins are inserted 2.5 centimeters (1 inch) into the pant. |
| F. | Using the tensile frame pushbutton controls for crosshead position, move pins apart until the load applied to the waistband is between 0 and 65 g. |
| G. | Click on RUN button. The test will start automatically. |
| H. | When the test is done, click on either FILE to save the data and graphs or NEXT to save only the data. |
| I. | Remove the sample from the pins. |
| J. | Repeat steps B, C and E through I for each waist specimen until the testing is complete. |
| K. | Using the tensile frame pushbutton controls for crosshead position, move pins toward one another so that the pant can be mounted on the pins without stretching the pant. |
| L. | Place the hip section of a fresh sample (not used for waistband testing) onto the knurled section of the top pin. Center one side of the pant on the top of the pin. Use a single binder clip to hold the pant in place on the pin; do not stretch the pant during application of the clip. |
| M. | Click on ZERO to the tare the load of the pant. Only tare the weight of the first pant for each sample population, not for each specimen. |
| N. | Place the hip section on the opposite side of the pant on the bottom pin and clip in place as for the first pin. Adjust pant so both top and bottom pins are inserted 2.5 centimeters (1 inch) into the pant. |
| O. | Using the tensile frame pushbutton controls for crosshead position, move pins apart until the load applied to the hip section is between 0 and 65 g. |
| P. | Click on RUN button. The test will start automatically. |
| Q. | When the test is done, click on either FILE to save the data and graphs or NEXT to save only the data. |
| R. | Remove the sample from the pins. |
| S. | Repeat steps K, L and N through R for each hip specimen until the testing is complete. |

The circumference of a measured waistband or hip section at any tension may be calculated by multiplying the gage length at that tension by 2, and adding one half the circumference of the upper pin and one half the circumference of the lower pin. The waistband-to-hip circumference ratio is calculated by dividing the average circumference of the waistband at a given tension or load by the average circumference of the hip section at the same tension or load. At a load of 70 grams, for example, the WHCR for a pant is the average initial waistband circumference divided by the average initial hip circumference. Similarly, the WHCR for a pant at 2,000 grams is the average final waistband circumference divided by the average final hip circumference.

It will be appreciated that details of the foregoing embodiments, given for purposes of illustration, are not to be construed as limiting the scope of this invention. Although only a few exemplary embodiments of this invention have been described in detail above, those skilled in the art will readily appreciate that many modifications are possible in the exemplary embodiments without materially departing from the novel teachings and advantages of this invention. Accordingly, all such modifications are intended to be included within the scope of this invention, which is defined in the following claims and all equivalents thereto. Further, it is recognized that many embodiments may be conceived that do not achieve all of the advantages of some embodiments, particularly of the preferred embodiments, yet the absence of a particular advantage shall not be construed to necessarily mean that such an embodiment is outside the scope of the present invention.

## Claims

1. A process for making a prefastened and refastenable pant (20), comprising:
providing a plurality of discrete articles, each article having first and second waist regions (22, 24), a crotch region (26) interconnecting the waist regions (22, 24), a longitudinal centerline (308), first and second fastening components (82, 83, 84, 85) disposed in the first and second waist regions (22, 24) respectively and adapted to refastenably engage one another, the first waist region (22) having opposed side panels (34));
providing an activatable retractive material in at least one of the waist regions (22, 24);
folding each article through the crotch region (26);
folding the opposed side panels (34) parallel to the longitudinal centerline (308) to overlap at least portions of the first and second fastening components (82, 83, 84, 85); and
engaging the first and second fastening components (82, 83, 84, 85);
**characterised in that** said process further comprises the step of:
activating at least a portion of the retractive material causing the retractive material to retract subsequent to engagement of the fastening components (82, 83, 84, 85).

2. The process of claim 1, wherein activating the retractive material comprises applying electromagnetic radiation.

3. The process of claim 1, wherein activating the retractive material comprises applying heat.

4. The process of claim 3, wherein a greater volume of heated air is applied to a waistband as compared to a hip section.

5. The process of claim 3, wherein a higher temperature air flow is applied to a waistband (75) as compared to a hip section (77).

6. The process of claim 1, further comprising temporarily maintaining the retractive material in an extended and unstable state by application of a compaction force.

7. The process of any preceding claim, wherein activating the retractive material provides a waistband-to-hip circumference ratio of about 95 percent or less.

8. The process of claim 7, wherein activating the retractive material provides a waist-to-hip circumference ratio of about 90 percent or less.

9. The process of claim 8, wherein activating the retractive material provides a waistband-to-hip circumference ratio of about 75 to about 90 percent.

10. An apparatus for making a prefastened and refastenable pant (20), comprising:
a pant assembly unit (200) adapted to provide a plurality of discrete articles, each article having first and second waist regions (22, 24), a crotch region (26) interconnecting the waist regions (22, 24), a longitudinal centerline (308), first and second fastening components (82, 83, 84, 85) disposed in the first and second waist regions (22, 24) respectively and adapted to refastenably engage one another, the first waist region (22) having opposed side panels (34), the pant assembly unit (200) further adapted to provide an activatable retractive material in at least one of the waist regions (22, 24);
a product folding mechanism (202) adapted to fold each article through the crotch region (26);
a side panel folding mechanism (202) adapted to fold the opposed side panels (34) parallel to the longitudinal centerline (308) to overlap at least portions of the first and second fastening components (82, 83, 84, 85); and
a fastener engaging mechanism (204) adapted to engage the first and second fastening components (82, 83, 84, 85);
**characterised in that** said apparatus further comprises:
an activating mechanism (206) adapted to activate at least a portion of the retractive material and cause the retractive material to retract subsequent to engagement of the fastening components (82, 83, 84, 85).

11. The apparatus of claim 10, wherein the activating mechanism (206) emits electromagnetic radiation.

12. The apparatus of claim 10, wherein the activating mechanism (206) emits heated air.

13. The apparatus of claim 10, wherein a greater volume of heated air is applied to a waistband (75) as compared to a hip section (77).

14. The apparatus of claim 10, wherein a higher temperature air flow is applied to a waistband (75) as compared to a hip section (77).

15. A prefastened and refastenable pant (20), comprising:
a chassis (32) defining a first waist region (22) having opposed side panels (34), an opposite second waist region (24), a crotch region (26) disposed between and interconnecting the waist regions (22, 24), and a longitudinal centerline (308), the waist regions (22, 24) together defining a waistband (75) and a hip section (77);
at least one first fastening component (82, 83, 84, 85) disposed in the first waist region (22);
at least one second fastening component disposed in the second waist region and adapted to refastenably engage the first fastening component;
a retractive material disposed in at least the waistband;
wherein the pant (20) is folded through the crotch region (26) and folded through the opposed side panels (34) so that portions of the waist regions (22, 24) overlap, the first and second fastening components (82, 83, 84, 85) are engaged with one another to maintain the pant (20) in a prefastened condition;
**characterised in that**:
the pant (20) has a waistband-to-hip circumference ratio, as determined using the test-method described herein at a 70 gram loading, of about 95 percent or less which results from activation of the retractive material after the fastening components (82, 83, 84, 85) are engaged to one another.

16. The prefastened and refastenable pant (20) of claim 15, wherein the pant (20) has a waistband-to-hip circumference ratio of about 90 percent or less.

17. The prefastened and refastenable pant (20) of claim 16, wherein the pant (20) has a waistband-to-hip circumference ratio of about 75 to about 90 percent.

18. The prefastened and refastenable pant (20) of claim 15, wherein the pant (20) has a waistband-to-hip circumference ratio measured at 2000 grams of about 100 percent.

19. The prefastened and refastenable pant (20) of any of claims 15 to 18, wherein the retractive material comprises an elastomeric material.

20. The prefastened and refastenable pant (20) of any of claims 15 to 18, wherein the retractive material comprises a nonelastomeric material.

21. The prefastened and refastenable pant (20) of any of claims 15 to 20, wherein the retractive material is disposed in the side panel (34).

## Patentansprüche

1. Verfahren zum Herstellen einer vorgeschlossenen und wiederverschließbaren Hose (20), umfassend:
Bereitstellen einer Vielzahl von diskreten Artikeln, wobei jeder Artikel einen ersten und zweiten Taillenbereich (22, 24), einen Schrittbereich (26), der die Taillenbereiche (22, 24) verbindet, eine Längsmittellinie (308) und erste und zweite Verschlusskomponenten (82, 83, 84, 85) aufweist, die in dem ersten beziehungsweise zweiten Taillenbereich (22, 24) angeordnet sind und geeignet sind, sich wiederverschließbar miteinander zu verbinden, wobei der erste Taillenbereich (22) gegenüberliegende Seitenbahnen (34) aufweist;
Bereitstellen eines aktivierbaren, rückziehbaren Materials in mindestens einem der Taillenbereiche (22, 24);
Falten jedes Artikels durch den Schrittbereich (26);
Falten der gegenüberliegenden Seitenbahnen (34) parallel zu der Längsmittellinie (308), um zumindest mit Teilen der ersten und zweiten Verschlusskomponente (82, 83, 84, 85) zu überlappen; und
In Verbindung Bringen der ersten und zweiten Verschlusskomponente (82, 83, 84, 85);
**dadurch gekennzeichnet, dass** das Verfahren des Weiteren den Schritt umfasst:
Aktivieren zumindest eines Teils des rückziehbaren Materials, welches das rückziehbare Material dazu bringt, sich zurückzuziehen, nachfolgend dem in Verbindung Bringen der Verschlusskomponenten (82, 83, 84, 85).

2. Verfahren gemäß Anspruch 1, wobei das Aktivieren des rückziehbaren Materials das Anwenden von elektromagnetischer Strahlung umfasst.

3. Verfahren gemäß Anspruch 1, wobei das Aktivieren des rückziehbaren Materials das Anwenden von Wärme umfasst.

4. Verfahren gemäß Anspruch 3, wobei im Vergleich zu einem Hüftabschnitt ein größeres Volumen von erwärmter Luft auf einen Taillenbund angewendet wird.

5. Verfahren gemäß Anspruch 3, wobei im Vergleich zu einem Hüftabschnitt (77) ein Luftstrom mit höherer Temperatur auf einen Taillenbund (75) angewendet wird.

6. Verfahren gemäß Anspruch 1, welches des Weiteren das zeitweise Erhalten des rückziehbaren Materials in einem gedehnten und instabilen Zustand durch Anwenden einer Verdichtungskraft umfasst.

7. Verfahren gemäß einem der vorherigen Ansprüche, wobei das Aktivieren des rückziehbaren Materials ein Taillenbund-zu-Hüfte Umfangsverhältnis von ungefähr 95 Prozent oder weniger bereitstellt.

8. Verfahren gemäß Anspruch 7, wobei das Aktivieren des rückziehbaren Materials ein Taillenbund-zu-Hüfte Umfangsverhältnis von ungefähr 90 Prozent oder weniger bereitstellt.

9. Verfahren gemäß Anspruch 8, wobei das Aktivieren des rückziehbaren Materials ein Taillenbund-zu-Hüfte Umfangsverhältnis von ungefähr 75 bis ungefähr 90 Prozent bereitstellt.

10. Gerät zum Herstellen einer vorgeschlossenen und wiederverschließbaren Hose (20), umfassend:
eine Hosenzusammensetzeinheit (200), welche geeignet ist, eine Vielzahl von diskreten Artikeln bereitzustellen, wobei jeder Artikel einen ersten und zweiten Taillenbereich (22, 24), einen Schrittbereich (26), der die Taillenbereiche (22, 24) verbindet, eine Längsmittellinie (308) und erste und zweite Verschlusskomponenten (82, 83, 84, 85) aufweist, die in dem ersten beziehungsweise zweiten Taillenbereich (22, 24) angeordnet sind und geeignet sind, sich wiederverschließbar miteinander zu verbinden, wobei der erste Taillenbereich (22) gegenüberliegende Seitenbahnen (34) aufweist, wobei die Hosenzusammensetzeinheit (200) des Weiteren geeignet ist, ein aktivierbares, rückziehbares Material in mindestens einem der Taillenbereiche (22, 24) bereitzustellen;
einen Produktfaltmechanismus (202), welcher geeignet ist, jeden Artikel durch den Schrittbereich (26) zu falten;
einen Seitenbahnfaltmechanismus (202), welcher geeignet ist, gegenüberliegende Seitenbahnen (34) parallel zu der Längsmittellinie (308) zu falten, um zumindest mit Teilen der ersten und zweiten Verschlusskomponente (82, 83, 84, 85) zu überlappen; und
einen Verschlussverbindemechanismus (204), welcher geeignet ist, die erste und zweite Verschlusskomponente (82, 83, 84, 85) in Verbindung zu bringen; **dadurch gekennzeichnet, dass** das Gerät des Weiteren umfasst:
einen Aktivierungsmechanismus (206), welcher geeignet ist, zumindest einen Teil des rückziehbaren Materials zu aktivieren und das rückziehbare Material dazu zu bringen, sich zurückzuziehen nachfolgend dem in Verbindung Bringen der Verschlusskomponenten (82, 83, 84, 85).

11. Gerät gemäß Anspruch 10, wobei der Aktivierungsmechanismus (206) elektromagnetische Strahlung emittiert.

12. Gerät gemäß Anspruch 10, wobei der Aktivierungsmechanismus (206) erwärmte Luft emittiert.

13. Gerät gemäß Anspruch 10, wobei im Vergleich zu einem Hüftabschnitt (77) ein größeres Volumen von erwärmter Luft auf einen Taillenbund (75) angewendet wird.

14. Gerät gemäß Anspruch 3, wobei im Vergleich zu einem Hüftabschnitt (77) ein Luftstrom mit höherer Temperatur auf einen Taillenbund (75) angewendet wird.

15. Vorgeschlossene und wiederverschließbare Hose (20), umfassend:
einen Rahmen (32), der einen ersten Taillenbereich (22) mit gegenüberliegenden Seitenbahnen (34), einen gegenüberliegenden zweiten Taillenbereich (24), einen Schrittbereich (26), der zwischen den Taillenbereichen (22, 24) angeordnet ist und diese verbindet, und eine Längsmittellinie (308) definiert, wobei die Taillenbereiche (22, 24) zusammen einen Taillenbund (75) und einen Hüftabschnitt (77) definieren;
mindestens eine erste Verschlusskomponente (82, 83, 84, 85), die in dem ersten Taillenbereich (22) angeordnet ist;
mindestens eine zweite Verschlusskomponente, die in dem zweiten Taillenbereich angeordnet ist und geeignet ist, sich wiederverschließbar mit der ersten Verschlusskomponente zu verbinden;
ein rückziehbares Material, welches zumindest in dem Taillenbund angeordnet ist;
wobei die Hose (20) durch den Schrittbereich (26) gefaltet ist und durch die gegenüberliegenden Seitenbahnen (34) gefaltet ist, so dass Teile der Taillenbereiche (22, 24) überlappen, wobei die erste und zweite Verschlusskomponente (82, 83, 84, 85) miteinander verbunden sind, um die Hose (20) In einem vorgeschlossenen Zustand zu halten;
**dadurch gekennzeichnet, dass**:
die Hose (20) ein Taillenbund-zu-Hüfte Umfangsverhältnis, wie unter Verwendung des Testverfahrens wie hierin beschrieben bei einer 70 Gramm Last bestimmt, von ungefähr 95 Prozent oder weniger aufweist, was durch die Aktivierung des rückziehbaren Materials, nachdem die Verschlusskomponenten (82, 83, 84, 85) miteinander in Verbindung gebracht sind, verursacht ist.

16. Vorgeschlossene und wiederverschließbare Hose (20) gemäß Anspruch 15, wobei die Hose (20) ein Taillenbund-zu-Hüfte Umfangsverhältnis von ungefähr 90 Prozent oder weniger aufweist.

17. Vorgeschlossene und wiederverschließbare Hose (20) gemäß Anspruch 16, wobei die Hose (20) ein Taillenbund-zu-Hüfte Umfangsverhältnis von ungefähr 75 bis ungefähr 90 Prozent aufweist.

18. Vorgeschlossene und wiederverschließbare Hose (20) gemäß Anspruch 15, wobei die Hose (20) ein Taillenbund-zu-Hüfte Umfangsverhältnis, gemessen bei 2000 Gramm aufweist, von ungefähr 100 Prozent.

19. Vorgeschlossene und wiederverschließbare Hose (20) gemäß einem der Ansprüche 15 bis 18, wobei das rückziehbare Material ein elastomeres Material umfasst.

20. Vorgeschlossene und wiederverschließbare Hose (20) gemäß einem der Ansprüche 15 bis 18, wobei das rückziehbare Material ein nicht elastomeres Material umfasst.

21. Vorgeschlossene und wiederverschließbare Hose (20) gemäß einem der Ansprüche 15 bis 20, wobei das rückziehbare Material in der Seitenbahn (34) angeordnet ist.

## Revendications

1. Procédé pour fabriquer une culotte préattachée et rattachable (20), comprenant :
la fourniture d'une pluralité d'articles discrets, chaque article ayant des première et seconde régions de taille (22, 24), une région d'entrejambe (26) reliant mutuellement les régions de taille (22, 24), une ligne médiane longitudinale (308), des premier et second composants d'attache (82, 83, 84, 85) disposés respectivement dans les première et seconde régions de taille (22, 24) et adaptés pour une prise rattachable l'un avec l'autre, la première région de taille (22) ayant des panneaux latéraux opposés (34) ;
la fourniture d'un matériau rétractif activable dans au moins l'une des régions de taille (22, 24) ;
le pliage de chaque article à travers la région d'entrejambe (26) ;
le pliage des panneaux latéraux opposés (34) parallèlement à la ligne médiane longitudinale (308) pour recouvrir au moins des parties des premier et second composants d'attache (82, 83, 84, 85) ; et
la mise en prise des premier et second composants d'attache (82, 83, 84, 85) ;
**caractérisé en ce que** ledit procédé comprend en outre l'étape de :
activation d'au moins une partie du matériau rétractif en amenant le matériau rétractif à se rétracter postérieurement à la mise en prise des composants d'attache (82, 83, 84, 85).

2. Procédé selon la revendication 1, dans lequel l'activation du matériau rétractif comprend l'application d'un rayonnement électromagnétique.

3. Procédé selon la revendication 1, dans lequel l'activation du matériau rétractif comprend l'application de chaleur.

4. Procédé selon la revendication 3, dans lequel un plus grand volume d'air chauffé est appliqué à une ceinture par comparaison à une section de hanche.

5. Procédé selon la revendication 3, dans lequel un écoulement d'air à température plus élevée est appliqué à une ceinture (75) par comparaison à une section de hanche (77).

6. Procédé selon la revendication 1, comprenant en outre le maintien temporaire du matériau rétractif dans un état étendu et instable par application d'une force de compactage.

7. Procédé selon l'une quelconque des revendications précédentes, dans lequel l'activation du matériau rétractif fournit un rapport de circonférence de ceinture à hanche d'environ 95 pour-cent ou moins.

8. Procédé selon la revendication 7, dans lequel l'activation du matériau rétractif fournit un rapport de circonférence de taille à hanche d'environ 90 pour-cent ou moins.

9. Procédé selon la revendication 8, dans lequel l'activation du matériau rétractif fournit un rapport de circonférence de ceinture à hanche d'environ 75 à environ 90 pour-cent.

10. Appareil pour fabriquer une culotte préattachée et rattachable (20), comprenant :
une unité d'assemblage de culottes (200) adaptée pour fournir une pluralité d'articles discrets, chaque article ayant des première et seconde régions de taille (22, 24), une région d'entrejambe (26) reliant mutuellement les régions de taille (22, 24), une ligne médiane longitudinale (308), des premier et second composants d'attache (82, 83, 84, 85) disposés respectivement dans les première et seconde régions de taille (22, 24) et adaptés pour une prise rattachable l'un avec l'autre, la première région de taille (22) ayant des panneaux latéraux opposés (34), l'unité d'assemblage de culottes (200) étant en outre adaptée pour fournir un matériau rétractif activable dans au moins l'une des régions de taille (22, 24) ;
un mécanisme de pliage de produit (202) adapté pour plier chaque article à travers la région d'entrejambe (26) ;
un mécanisme de pliage de panneaux latéraux (202) adapté pour plier les panneaux latéraux opposés (34) parallélement à la ligne médiane longitudinale (308) pour recouvrir au moins des parties des premier et second composants d'attache (82, 83, 84, 85) ; et
un mécanisme de prise d'attache (204) adapté pour mettre en prise les premier et second composants d'attache (82, 83, 84, 85) ;
**caractérisé en ce que** ledit appareil comprend en outre :
un mécanisme d'activation (206) adapté pour activer au moins une partie du matériau rétractif et amener le matériau rétractif à se rétracter postérieurement à la mise en prise des composants de attache (82, 83, 84, 85).

11. Appareil selon la revendication 10, dans lequel le mécanisme d'activation (206) émet un rayonnement électromagnétique.

12. Appareil selon la revendication 10, dans lequel le mécanisme d'activation (206) émet de l'air chauffé.

13. Appareil selon la revendication 10, dans lequel un plus grand volume d'air chauffé est appliqué à une ceinture (75) par comparaison à une section de hanche (77).

14. Appareil selon la revendication 10, dans lequel un écoulement d'air à température plus élevée est appliqué à une ceinture (75) par comparaison à une section de hanche (77).

15. Culotte préattachée et rattachable (20), comprenant :
un châssis (32) définissant une première région de taille (22) ayant des panneaux latéraux opposés (34), une seconde région de taille opposée (24), une région d'entrejambe (26) disposée entre les régions de taille (22, 24) et reliant mutuellement celles-ci, et une ligne médiane longitudinale (308), les régions de taille (22, 24) définissant ensemble une ceinture (75) et une section de hanche (77) ;
au moins un premier composant d'attache (82, 83, 84, 85) disposé dans la première région de taille (22) ;
au moins un second composant d'attache disposé dans la seconde région de taille et adapté pour venir en prise de manière rattachable avec le premier composant de attache ;
un matériau rétractif disposé dans au moins la ceinture ;
dans laquelle la culotte (20) est pliée à travers la région d'entrejambe (26) et pliée à travers les panneaux latéraux opposés (34) de sorte que des parties des régions de taille (22, 24) se recouvrent, les premier et second composants d'attache (82, 83, 84, 85) sont en prise l'un avec l'autre pour maintenir la culotte (20) dans un état préattaché ;
**caractérisée en ce que** :
la culotte (20) a un rapport de circonférence de ceinture à hanche, tel que déterminé en utilisant la méthode d'essai décrite ici à un chargement de 70 grammes, d'environ 95 pour-cent ou moins qui résulte de l'activation du matériau rétractif après que les composants de attache (82, 83, 84, 85) ont été mis en prise l'un avec l'autre.

16. Culotte préattachée et rattachable (20) selon la revendication 15, dans laquelle la culotte a un rapport de circonférence de ceinture à hanche d'environ 90 pour-cent ou moins.

17. Culotte préattachée et rattachable (20) selon la revendication 16, dans laquelle la culotte (20) a un rapport de circonférence de ceinture à hanche d'environ 75 à environ 90 pour-cent.

18. Culotte préattachée et rattachable (20) selon la revendication 15, dans laquelle la culotte (20) a un rapport de circonférence de ceinture à hanche mesuré à 2 000 grammes d'environ 100 pour-cent.

19. Culotte préattachée et rattachable (20) selon l'une quelconque des revendications 15 à 18, dans laquelle le matériau rétractif comprend un matériau élastomère.

20. Culotte préattachée et rattachable (20) selon l'une quelconque des revendications 15 à 18, dans laquelle le matériau rétractif comprend un matériau non élastomère.

21. Culotte préattachée et rattachable (20) selon l'une quelconque des revendications 15 à 20, dans laquelle le matériau rétractif est disposé dans le panneau latéral (34).
